# EUROPEAN PATENT SPECIFICATION

(11) **EP 4 356 137 B1**
(45) Date of publication and mention of the grant of the patent: **23.07.2025**
(21) Application number: 22735384.4
(22) Date of filing: 17.06.2022
(51) Int. Cl.: G01N 33/574

(54) **COLLAGEN TYPE XX ASSAY**
KOLLAGEN-TYP-XX-TEST
DOSAGE DE COLLAGÈNE DE TYPE XX

(30) Priority: 18.06.2021 GB 202108802
(43) Date of publication of application: 24.04.2024
(73) Proprietor: Nordic Bioscience A/S, 2730 Herlev (DK)
(72) Inventor: THORLACIUS-USSING, Jeppe, 2730 Herlev (DK); WILLUMSEN, Nicholas, 2730 Herlev (DK); KARSDAL, Morten, Asser, 2730 Herlev (DK)
(74) Representative: Beck Greener LLP
(86) International application number: PCT/EP2022/066635
(87) International publication number: WO 2022/263675

(56) References cited:
- WO-A1-2011/051278
- CN-A- 108 546 760
- THORLACIUS-USSING JEPPE ; ET AL: "Citation: Thorlacius-Ussing, Type XX Collagen Is Elevated in Circulation of Patients with Solid Tumors", INTERNATIONAL JOURNAL OF MOLECULAR SCIENCES, vol. 23, no. 8, 8 April 2022 (2022-04-08), pages 1 - 17, XP055956696, Retrieved from the Internet <URL:https://www.mdpi.com/1422-0067/23/8/4144/htm>

## Description

### Field of the Invention

The present invention relates to immunoassays, in particular immunoassays for detecting and/or monitoring cancer in a patient, and to monoclonal antibodies and immunoassay kits for use in carrying out said immunoassays.

### Background

Cancer is a major global health problem and the burden of cancer incidence and mortality is rising worldwide [1]. One part of the problem is the lack of relevant biomarker tools that can, amongst other things, detect cancer earlier and predict response to treatment. Further, traditional biomarkers usually involve invasive procedures such as tissue biopsies [2]. A promising alternative to these approaches is liquid biopsies. In recent years, the tumor microenvironment surrounding the cancer cells has been given more attention and is a prime place to look for non-invasive biomarkers.

In particular, the extracellular matrix, defined as the non-cellular part of tissues has been recognized as an important part of cancer development [3]. The dominant ECM proteins are the collagens, which are important for tumor stiffness, tumor immunity and cancer metastasis [4]. In cancer, the dynamic balance of ECM formation and degradation is knocked askew. Cells in the tumor microenvironment influence the remodelling of collagen and collagen reciprocally influences the behaviour of cells [4,5]. Collagens such as type IV have well-described expression patterns, localization and function in the cancer context. It is ubiquitous in basement membranes and serves as a barrier for invading tumor cells, one that can be broken down to facilitate metastasis [6,7]. However, most of the cancer research into collagens has focused on the abundant and well-characterized collagens, such as type I, III or IV collagens, whereas many of the more poorly characterized collagens remain unexplored.

Type XX collagen is one such unexplored collagen. Based on its structural features, type XX collagen is part of the family of fibril-associated collagens with interrupted helices (FACITs). This family of collagens is thought to associate with the fibrillar collagens to regulate their organization and interactions [8]. Structural features of type XX collagen include several fibronectin type III repeats, a von Willebrand factor A domain, a thrombospondin-like domain as well as collagenous triple-helix domains (G-X-Y) interspersed with non-collagenous domains [8]. Within the FACIT family, type XII and XIV are the closest relatives to type XX collagen since they are the only FACITs to also have fibronectin domains [8].

Only a little is known about the expression, localization and function of type XX collagen. It was originally cloned from chick embryos in which expression was mostly limited to corneal epithelium, but detectable in embryonic skin, lung, sternal cartilage, and tendon [8]. Several authors have pointed out the parallels between cancer and development [9,10]. RNA expression data from the human protein atlas (https://www.proteinatlas.org/ENSG00000101203-COL20A1/tissue) also suggests an enrichment of COL20A1 expression in human brain and comparatively minor upticks in testis and spleen tissues. A role in the brain is also confirmed by data from the cancer genome atlas (TCGA) initiative wherein COL20A1 levels are comparatively elevated in glioma tissues (https://www.proteinatlas.org/ENSG00000101203-COL20A1/pathology). Thus, looking strictly at expression levels, type XX collagen seems to play a role in brain function and in brain cancers. From this perspective, type XX collagen does not seem to be present in appreciable amounts in other tissues.

Despite the apparent scarcity of type XX collagen expression, COL20A1 has been described in the cancer literature. Reports usually describe COL20A1 at the DNA or RNA level and usually in broad and general screenings. In line with the brain association described above and based on cDNA microarrays, COL20A1 was elevated in so-called brain-tumor initiating cells versus regular glioma cell lines and normal brain astrocytes [12]. In a separate study, using biopsy-derived glioma cell models, a downregulation of COL20A1 RNA was observed after treatment with histone deacetylase inhibitors [13]. In other cancers, reports on type XX collagen are rare, but COL20A1 was included in a 16-gene signature associated with breast cancer recurrence, metastasis and poor survival in a Chinese population [11]. The non-coding RNA Lnc-COL20A1-2 was described in CN108546760 as a pancreatic cancer prognosis molecular marker. And COL20A1 is reportedly also upregulated in early-stage prostate tumorigenesis [14]. WO2011/051278 describes the use of COL XX (COL20A1) as a tumour biomarker for the detection of lung cancer. Notably, these screenings do not follow up with any functional studies on type XX collagen, so insight into its function and distribution is still sorely lacking.

### Summary

The applicant has now developed an enzyme-linked immunosorbent assay (ELISA) to quantify the presence of a biomarker (referred to herein as "PRO-C20") of type XX collagen, in blood. The assay was optimized and validated, and used to determine the levels of circulating type XX collagen in the serum of cancer patients and healthy controls. It was found that the assay was robust, with specificity for type XX collagen and with sensitivity to detect levels in both healthy controls and patients with disease (cancer). The data shows that PRO-C20 levels were significantly elevated in the serum of cancer patients compared to healthy controls. The data also shows that, in patients with cancer, high levels of PRO-C20 are associated with poor overall survival rates.

The invention is defined by the appended claims.

Accordingly, in a first aspect, the present invention provides a method of immunoassay, the method comprising:
(i) contacting a sample from a patient with a monoclonal antibody that specifically binds to a C-terminus amino acid sequence QGASTQGLWE (SEQ ID NO: 1) (which C-terminus amino acid sequence is referred to herein as "PRO-C20" and/or the "target sequence", peptides consisting of or comprising said C-terminus amino acid sequence also being referred to herein as "PRO-C20" and/or the "target peptide"); and
(ii) detecting and determining the amount of binding between said monoclonal antibody and peptides in the sample.

The method is preferably a method of immunoassay for detecting and/or monitoring a disease in a patient and/or assessing the severity of a disease in a patient. The method preferably further comprises:
(iii) correlating said amount of binding of said monoclonal antibody as determined in step (ii) with values associated with normal healthy subjects, and/or with values associated with known disease severity, and/or with values obtained from said patient at a previous time point, and/or with a predetermined cut-off value.

In preferred embodiments, the disease is cancer. The cancer may for example be bladder cancer, breast cancer, colorectal cancer, head and neck cancer, kidney cancer, liver cancer, lung cancer, melanoma, ovarian cancer, pancreatic cancer, prostate cancer, or stomach cancer. The disease may in particular be pancreas ductal adenocarcinoma (PDAC).

As noted above, the method may in certain embodiments be a method for assessing the severity of a disease, such as for example cancer, in a patient. For example, the method may be a method for determining the prognosis of a cancer in a patient, such as for example determining the likely survival time and/or probability of survival of the patient.

In preferred embodiments, the monoclonal antibody does not specifically bind to an elongated version of the target sequence which is QGASTQGLWES (SEQ ID NO: 2) (i.e. a version of the PRO-C20 target sequence extended at its C-terminus by the addition of a serine residue). Preferably, the ratio of the affinity of said antibody for the PRO-C20 target sequence to the affinity of said antibody for the elongated version of the target sequence is at least 10 to 1, and more preferably is at least 20 to 1 or at least 30 to 1.

In preferred embodiments, the monoclonal antibody does not specifically bind to a truncated version of the target sequence which is QGASTQGLW (SEQ ID NO: 3) (i.e. a version of the PRO-C20 target sequence truncated by removal of the final glutamic acid residue). Preferably, the ratio of the affinity of said antibody for the PRO-C20 target sequence to the affinity of said antibody for the truncated version of the target sequence is at least 10 to 1, and more preferably is at least 20 to 1 or at least 30 to 1.

Preferably, the monoclonal antibody is a monoclonal antibody that is raised against a synthetic peptide having the C-terminus amino acid sequence QGASTQGLWE (SEQ ID NO: 1).

The sample is preferably a biofluid. The biofluid may be, but is not limited to, blood, serum, plasma, urine or a supernatant from cell or tissue cultures. Preferably the biofluid is blood, serum or plasma.

The immunoassay may be, but is not limited to, a competition assay or a sandwich assay. The immunoassay may, for example, be a radioimmunoassay or an enzyme-linked immunosorbent assay (ELISA). Such assays are techniques known to the person skilled in the art.

As used herein the term "amount of binding" refers to the quantification of binding between the monoclonal antibody and peptides in the patient sample. Said quantification may for example be determined by comparing the measured values of binding in the patient sample against a calibration curve produced using measured values of binding in standard samples containing known concentrations of a peptide to which the antibody specifically binds, in order to thereby determine the quantity of peptide to which the antibody specifically binds in the patient sample. In the Examples set out below, an ELISA method is used in which spectrophotometric analysis is used to measure the amount of binding both in the patient samples and when producing the calibration curve. However, any suitable analytical method can be used.

As used herein the term "predetermined cut-off value" means an amount of binding that is determined statistically to be indicative of a high likelihood of a disease or a particular severity thereof in a patient, in that a measured value of the target peptide in a patient sample that is at or above the statistical cut-off value corresponds to at least a 70% probability, preferably at least an 75% probability, more preferably at least an 80% probability, more preferably at least an 85% probability, more preferably at least a 90% probability, and most preferably at least a 95% probability of the presence of said disease or said particular severity thereof. For example, in patients with PDAC, a PRO-C20 level of 2.59 nM may in certain embodiments be used as a predetermined cut-off value indicating that the severity of the cancer is such that the patient has about a high (e.g. about 75%) probability of dying within the next 6 months, and/or has a very high (e.g. at least 95%) probability of dying within about the next 2 years.

As used herein, the term "values associated with normal healthy subjects" means standardised quantities of binding determined by the method described supra for samples from subjects considered to be healthy, i.e. without disease; and the term "values associated with known disease severity" means standardised quantities of binding determined by the method described supra for samples from patients known to have disease of a known severity.

As used herein the term "C-terminus" refers to a C-terminal peptide sequence at the extremity of a polypeptide, i.e. at the C-terminal end of the polypeptide, and is not to be construed as meaning in the general direction thereof.

As used herein, the terms "peptide" and "polypeptide" are used synonymously.

As used herein the term "monoclonal antibody" refers to both whole antibodies and to fragments thereof that retain the binding specificity of the whole antibody, such as for example a Fab fragment, F(ab')2 fragment, single chain Fv fragment, or other such fragments known to those skilled in the art. As is well known, whole antibodies typically have a "Y-shaped" structure of two identical pairs of polypeptide chains, each pair made up of one "light" and one "heavy" chain. The N-terminal regions of each light chain and heavy chain contain the variable region, while the C-terminal portions of each of the heavy and light chains make up the constant region. The variable region comprises three complementarity determining regions (CDRs), which are primarily responsible for antigen recognition. The constant region allows the antibody to recruit cells and molecules of the immune system. Antibody fragments retaining binding specificity comprise at least the CDRs and sufficient parts of the rest of the variable region to retain said binding specificity.

In the methods of the present invention, a monoclonal antibody comprising any constant region known in the art can be used. Human constant light chains are classified as kappa and lambda light chains. Heavy constant chains are classified as mu, delta, gamma, alpha, or epsilon, and define the antibody's isotype as IgM, IgD, IgG, IgA, and IgE, respectively. The IgG isotype has several subclasses, including, but not limited to IgGl, IgG2, IgG3, and IgG4. The monoclonal antibody may preferably be of the IgG isotype, including any one of IgGl, IgG2, IgG3 or IgG4.

The CDR of an antibody can be determined using methods known in the art such as that described by Kabat et al. Antibodies can be generated from B cell clones as described in the examples. The isotype of the antibody can be determined by ELISA specific for human IgM, IgG or IgA isotype, or human IgG1, IgG2, IgG3 or IgG4 subclasses. The amino acid sequence of the antibodies generated can be determined using standard techniques. For example, RNA can be isolated from the cells, and used to generate cDNA by reverse transcription. The cDNA is then subjected to PCR using primers which amplify the heavy and light chains of the antibody. For example primers specific for the leader sequence for all VH (variable heavy chain) sequences can be used together with primers that bind to a sequence located in the constant region of the isotype which has been previously determined. The light chain can be amplified using primers which bind to the 3' end of the Kappa or Lamda chain together with primers which anneal to the V kappa or V lambda leader sequence. The full length heavy and light chains can be generated and sequenced.

The monoclonal antibody or fragment thereof may preferably comprise one or more complementarity-determining regions (CDRs) selected from:
CDR-H1: DYSMH (SEQ ID NO: 11)
CDR-H2: WINTETGEPTYADGFKG (SEQ ID NO: 12)
CDR-H3: GPY
CDR-L1: RSSQSIVHNNGKIYLE (SEQ ID NO: 13)
CDR-L2: KVSNRFS (SEQ ID NO: 14)
CDR-L3: FQGSHVPYT (SEQ ID NO: 15)

Preferably the antibody or fragment thereof comprises at least 2,3,4,5 or 6 of the above listed CDR sequences.

Preferably the monoclonal antibody or fragment thereof has a light chain variable region comprising the CDR sequences:
CDR-L1: RSSQSIVHNNGKIYLE (SEQ ID NO: 13)
CDR-L2: KVSNRFS (SEQ ID NO: 14) and
CDR-L3: FQGSHVPYT (SEQ ID NO: 15)

Preferably the monoclonal antibody or fragment thereof has a light chain that comprises framework sequences between the CDRs, wherein said framework sequences are substantially identical or substantially similar to the framework sequences between the CDRs in the light chain sequence below (in which the CDRs are shown in bold and underlined, and the framework sequences are shown in italics):

RSSQSIVHNNGKIYLEWFLQKPGQSPKALIQKVSNRFSGVPDRFSGSGSGTDFTLKISRVEA
EDLGVYYCFQGSHVPYT (SEQ ID NO: 16)

Preferably the monoclonal antibody or fragment thereof has a heavy chain variable region comprising the CDR sequences:
CDR-H1: DYSMH (SEQ ID NO: 11)
CDR-H2: WINTETGEPTYADGFKG (SEQ ID NO: 12) and
CDR-H3: GPY

Preferably the monoclonal antibody or fragment thereof has a heavy chain that comprises framework sequences between the CDRs, wherein said framework sequences are substantially identical or substantially similar to the framework sequences between the CDRs in the heavy chain sequence below (in which the CDRs are shown in bold and underlined, and the framework sequences are shown in italics):

DYSMHWVKQAPGKGLKWMGWINTETGEPTYADGFKGRFAFSLETSASTAYLKINNLKNDDTA
TYFCARGPY (SEQ ID NO: 17)

As used herein, the framework amino acid sequences between the CDRs of an antibody are substantially identical or substantially similar to the framework amino acid sequences between the CDRs of another antibody if they have at least 70%, 80%, 90% or at least 95% similarity or identity. The similar or identical amino acids may be contiguous or non-contiguous.

The framework sequences may contain one or more amino acid substitutions, insertions and/or deletions. Amino acid substitutions may be conservative, by which it is meant the substituted amino acid has similar chemical properties to the original amino acid. A skilled person would understand which amino acids share similar chemical properties. For example, the following groups of amino acids share similar chemical properties such as size, charge and polarity: Group 1 Ala, Ser, Thr, Pro, Gly; Group 2 Asp, Asn, Glu, Gln; Group 3 His, Arg, Lys; Group 4 Met, Leu, Ile, Val, Cys; Group 5 Phe Thy Trp.

A program such as the CLUSTAL program to can be used to compare amino acid sequences. This program compares amino acid sequences and finds the optimal alignment by inserting spaces in either sequence as appropriate. It is possible to calculate amino acid identity or similarity (identity plus conservation of amino acid type) for an optimal alignment. A program like BLASTx will align the longest stretch of similar sequences and assign a value to the fit. It is thus possible to obtain a comparison where several regions of similarity are found, each having a different score. Both types of analysis are contemplated in the present invention. Identity or similarity is preferably calculated over the entire length of the framework sequences.

In certain preferred embodiments, the monoclonal antibody or fragment thereof may comprise the light chain variable region sequence: 
DVLLTQTPLSLPVSLGDQASISCRSSQSIVHNNGKIYLEWFLQKPGQSPKALIQKVSNRFSG
VPDRFSGSGSGTDFTLKISRVEAEDLGVYYCFQGSHVPYTFGGGTKLEIK (SEQ ID NO:
18)
 and/or the heavy chain variable region sequence: 
QIQLMQSGPELKKPGETVKISCKASGYPFTDYSMHWVKQAPGKGLKWMGWINTETGEPTYAD
GFKGRFAFSLETSASTAYLKINNLKNDDTATYFCARGPYWGQGTLVTVSA (SEQ ID NO:
19)
 (CDRs bold and underlined; Framework sequences in italics)

In a second aspect, the present invention provides a monoclonal antibody that specifically binds to a C-terminus amino acid sequence QGASTQGLWE (SEQ ID NO: 1). The monoclonal antibody is suitable for use in an immunoassay according to the first aspect of the invention, and preferred and other optional embodiments of the monoclonal antibody according to the second aspect of the invention will be apparent from the discussion *supra* of the monoclonal antibodies for use the in the first aspect of the invention and preferred and other optional embodiments thereof.

In a third aspect, the application relates to an immunoassay kit comprising a monoclonal antibody according to the second aspect, and at least one of;
- a streptavidin coated well plate;
- a biotinylated peptide Biotin-L- QGASTQGLWE (SEQ ID NO: 4), wherein L is an optional linker;
- a secondary antibody for use in a sandwich immunoassay;
- a calibrator protein comprising the C-terminus amino acid sequence QGASTQGLWE (SEQ ID NO: 1);
- an antibody biotinylation kit;
- an antibody HRP labelling kit;
- an antibody radiolabelling kit; and
- an assay visualisation kit.

### Figures

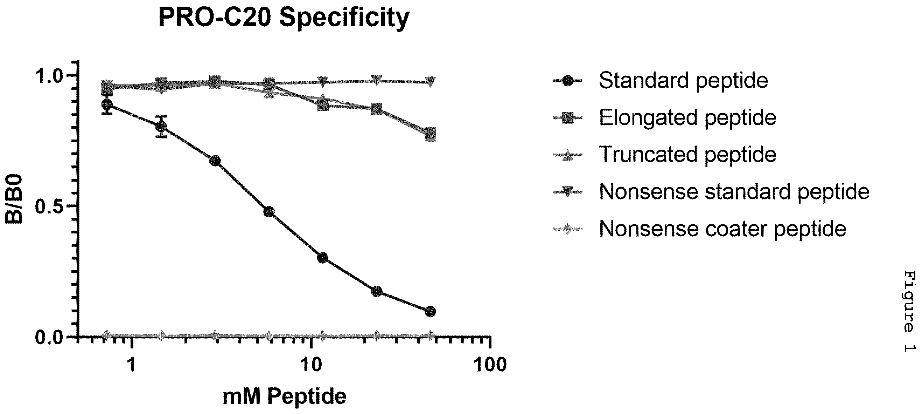

**Figure 1****:** Specificity test of the PRO-C20 assay. Inhibition curves for the standard peptide (RHLEGRGEPGAVGQMGSPGQQGASTQGLWE (SEQ ID NO: 6)), elongated peptide (QGASTQGLWES (SEQ ID NO: 2)), truncated peptide (QGASTQGLW (SEQ ID NO: 3)) as well as a non-sense standard peptide (SHAHQRTGGN (SEQ ID NO: 8)) and a non-sense coater peptide (Biotin-SHAHQRTGGN (SEQ ID NO: 9)). Peptides were serially diluted two-fold to evaluate their capability of competing for antibody binding. Signal (B) is shown as a fraction of the background absorbance (B0), which corresponds to assay buffer, as a function of the peptide concentration on a logarithmic scale. Error bars indicate standard deviation from duplicate measurements.

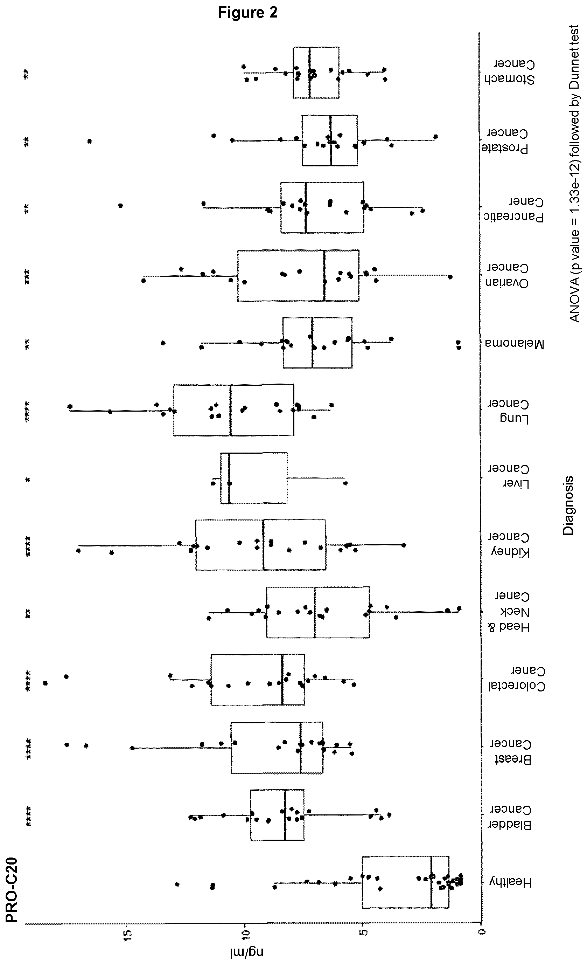

**Figure 2****:** PRO-C20 was measured in the serum of multiple cancer patient groups. Quantification of PRO-C20 in the serum of healthy controls (n = 33) and bladder cancer (n = 20), breast cancer (n = 20), colorectal cancer (n = 20), head & neck cancer (n = 20), kidney cancer (n = 20), liver cancer (n = 3), lung cancer (n = 20), melanoma (n = 20), ovarian cancer (n = 19), pancreatic cancer (n = 20), prostate cancer (n = 20) and stomach cancer (n = 20). PRO-C20 levels are presented as Tukey-style boxplots with data-point jitter: horizontal bars indicate the median; upper- and lower hinges of the box indicate the first and third quartiles (the 25th and 75th percentiles); whiskers extend from the hinges to the largest or smallest value but no further than 1.5*IQR (where IQR is the inter-quartile range between the first and third quartiles) in either the positive or negative direction. Samples measuring below the lower limit of measurement range (LLMR) were given the value of LLMR, as determined in the validation of PRO-C20. Differences in PRO-C20 levels between the cancer-groups and the healthy controls were evaluated by ordinary ANOVA followed by multiple comparisons to the controls with Dunnett test. **** indicates a p-value below 0.0001. *** below 0.001. ** below 0.01. * below 0.05.

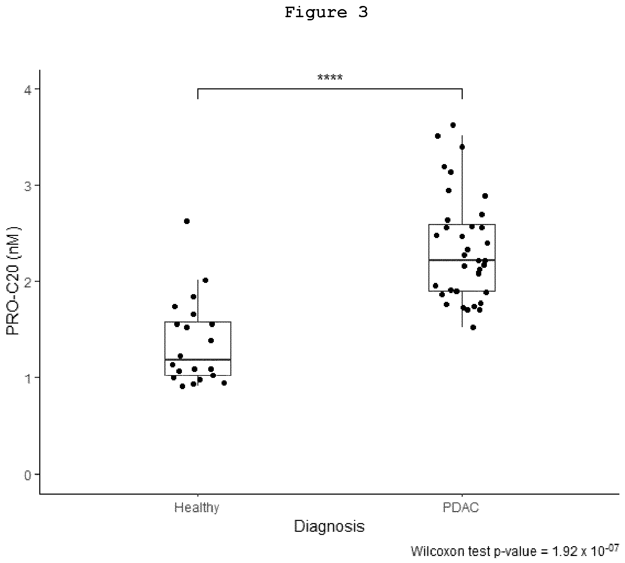

**Figure 3****:** Quantification of PRO-C20 in serum of healthy controls (n = 20) and patients with PDAC (n = 36).

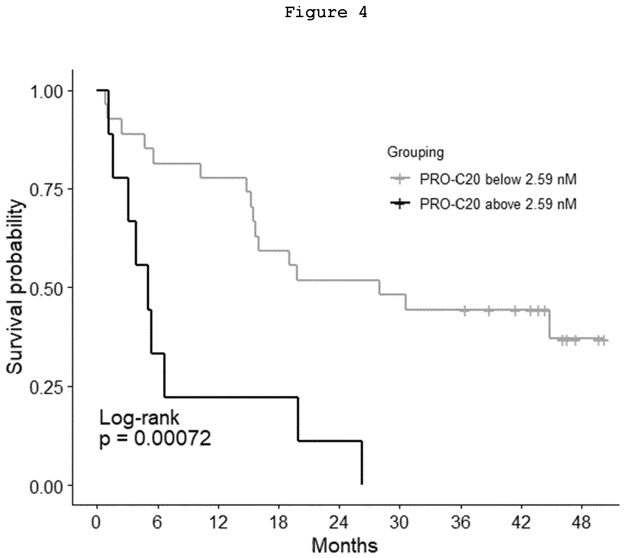

**Figure 4****:** Kaplan Meier curve evaluating the association between high PRO-C20 levels (above 2.59 nM) and overall survival in patients with PDAC.

### Examples

The presently disclosed embodiments are described in the following Examples, which are set forth to aid in the understanding of the disclosure, and should not be construed to limit in any way the scope of the disclosure as defined in the claims which follow thereafter. The following examples are put forth so as to provide those of ordinary skill in the art with a complete disclosure and description of how to make and use the described embodiments, and are not intended to limit the scope of the present disclosure nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### Materials and Methods

### Generation of Monoclonal Antibodies Targeting PRO-C20

A 10 amino-acid target peptide ¹²⁷⁵QGASTQGLWE¹²⁸⁴ (SEQ ID NO: 1), corresponding to the C-terminus of type XX collagen (UniprotKB: Q9P218) was purchased from Genscript (Piscataway, NJ, USA) and used for immunization.

More specifically, an immunogenic peptide (KLH-CGG-QGASTQGLWE (SEQ ID NO: 7)) was generated by covalently cross-linking the target peptide to Keyhole Limpet Hemocyanin (KLH) carrier protein using sulfosuccinimidyl 4-(N-maleimidomethyl) cyclohexane-1-carboxylate, SMCC (Thermo Scientific, Waltham, MA, USA, cat.no. 22322). Glycine and cysteine residues were added at the N-terminal end to ensure right linking of the carrier protein. Monoclonal antibodies were generated by subcutaneous immunization of six-week-old Balb/C mice with 200 µL emulsified antigen containing 100 µg immunogenic peptide mixed with Sigma Adjuvant System (Sigma cat. No. S6322). Consecutive immunizations were performed at 2-week intervals until stable sera titer levels were reached. The mouse with the highest titer was rested for four weeks and was then boosted with 100 µg immunogenic peptide in 100 µL 0.9% NaCl solution intravenously. Hybridoma cells were produced by fusing spleen cells with SP2/0 myeloma cells as previously described (Gefter, Margulies and Scharff, 1977). The resultant hybridoma cells were then cultured in 96-well microtiter plates and standard limited dilution was used to secure monoclonal growth.

The monoclonal antibodies were purified using protein-G-columns according to the manufacturer's instructions (GE Healthcare Life Sciences, Little Chalfont, UK, cat. #17-0404-01).

The best antibody clone for the biomarker was selected based on a preliminary competitive ELISA for the reactivity towards the selection peptide (the target peptide, QGASTQGLWE (SEQ ID NO: 1)), and not an elongated peptide (QGASTQGLWES (SEQ ID NO: 2)), a truncated peptide (QGASTQGLW (SEQ ID NO: 3)), and a non-sense KLH-conjugated peptide (IRQCPDRTYG-GGC-KLH (SEQ ID NO: 10)).

The antibody produced by the best performing clone was sequenced and the CDRs determined. The sequence of the chains are as follows (CDRs underlined and in bold, constant region italic) :
Heavy chain: Amino acid sequence (511 aa) 
QIQLMQSGPELKKPGETVKISCKASGYPFTDYSMHWVKQAPGKGLKWMGWINTETGEPTYAD
GFKGRFAFSLETSASTAYLKINNLKNDDTATYFCARGPYWGQGTLVTVSAAKTTAPSVYPLA
PVCGDTTGSSVTLGCLVKGYFPEPVTLTWNSGSLSSGVHTFPAVLQSDLYTLSSSVTVTSST
WPSQSITCNVAHPASSTKVDKKIEPRGPTIKPCPPCKCPAPNLLGGPSVFIFPPKIKDVLMI
SLSPIVTCVVVDVSEDDPDVQISWFVNNVEVHTAQTQTHREDYNSTLRVVSALPIQHQDWMS
GKEFKCKVNNKDLPAPIERTISKPKGSVRAPQVYVLPPPEEEMTKKQVTLTCMVTDFMPEDI
YVEWTNNGKTELNYKNTEPVLDSDGSYFMYSKLRVEKKNWVERNSYSCSVVHEGLHNHHTTK
SFSRTPGLDLDDVCAEAQDGELDGLWTTITIFISLFLLSVCYSASVTLFKVKWIFSSVVELK
QTISPDYRNMIGQGA (SEQ ID NO: 20)
Light chain: Amino acid sequence (219 aa)

DVLLTQTPLSLPVSLGDQASISCRSSQSIVHNNGKIYLEWFLQKPGQSPKALIQKVSNRFSG
VPDRFSGSGSGTDFTLKISRVEAEDLGVYYCFQGSHVPYTFGGGTKLEIKRADAAPTVSIFP
PSSEQLTSGGASVVCFLNNFYPKDINVKWKIDGSERQNGVLNSWTDQDSKDSTYSMSSTLTL
TKDEYERHNSYTCEATHKTSTSPIVKSFNRNEC (SEQ ID NO: 21)

### PRO-C20 ELISA Protocol

Several optimizations were made to the ELISA including the choice of assay buffer, incubation time and temperature as well as concentrations of antibody and peptides. The final PRO-C20 assay protocol was performed as follows: a 96-well streptavidin-coated ELISA plate was coated with 100 µL/well of 1.25 ng/mL biotinylated target peptide (Biotin-QGASTQGLWE (SEQ ID NO: 5)) peptide dissolved in assay buffer (25 mM TBS, 1% BSA (w/v), 0.1% Tween-20 (w/v), 2 g/L NaCl, pH 8.0) and incubated for 30 minutes at 20 °C with shaking at 300 RPM. After washing five times with washing buffer (25 mM Tris, 50 mM NaCl, pH 7.2), 20 µL/well of sample was added in duplicates followed by 100 µL/well of 50 ng/mL HRP-labelled monoclonal antibody in assay buffer and incubated for 1 hour at 20 °C with shaking at 300 RPM. After a second washing cycle, 100 µL/well of TMB was added and incubated for 15 minutes in darkness at 20 °C with shaking at 300 RPM. The reaction was stopped by adding 100 µL/well of 1% H₂SO₄. Absorbance was measured at 450 nm with 650 nm as reference. To generate a standard curve, 20 µL/well of 50 ng/mL standard peptide (RHLEGRGEPGAVGQMGSPGQQGASTQGLWE (SEQ ID NO: 6)), serially diluted two-fold, was added to appropriate wells and a four-parametric logistic regression model was used to fit a curve. Each plate included 5 quality control samples comprising one human serum, one horse serum, one human plasma and two peptide-in-assay-buffer samples to monitor intra- and inter-assay variation.

### Technical validation of the PRO-C20 ELISA

Antibody specificity was evaluated by the inhibition of signal by two-fold serial dilutions of the standard peptide (RHLEGRGEPGAVGQMGSPGQQGASTQGLWE (SEQ ID NO: 6)), elongated peptide (QGASTQGLWES (SEQ ID NO: 2)), truncated peptide (QGASTQGLW (SEQ ID NO: 3)) as well as a non-sense standard peptide (SHAHQRTGGN (SEQ ID NO: 8)) and a non-sense coater peptide (Biotin-SHAHQRTGGN (SEQ ID NO: 9)).

Linearity or parallelism was evaluated by two-fold serial dilutions of human serum samples and calculating the percentage recovery relative to the dilution. Accuracy was evaluated by spiking a known quantity of the standard peptide into a human serum sample and calculating the percentage recovery of the measured concentration of the spiked sample relative to the expected concentration of the non-spiked sample plus the known quantity spiked in. Similarly, accuracy was also evaluated by spiking one human serum sample into another human serum sample at different ratios (eg. 50:50 or 25:75) and calculating the percentage recovery relative to the sum of their separately quantified values.

The influence of commonly interfering substances including hemoglobin, lipids and biotin were evaluated by spiking human serum samples with a known quantity of the interfering substances (hemoglobin low = 2.5 mg/mL, high = 5 mg/mL; lipids low = 1.5 mg/mL, high = 5 mg/mL; biotin low = 5 ng/mL, high = 100 ng/mL) and calculating the percentage recovery relative to the non-spiked sample.

Assay variation was tested by running ten independent run of the assay using ten quality control samples in double determinations. Five of the quality control samples were human serum, one horse serum, one human plasma and three were standard peptide in assay-buffer of varying concentrations. Intra-assay variation was calculated as the mean coefficient of variance (CV%) between the double determinations on each of the ten runs. Inter-assay variation was calculated as the overall CV% for all determinations on the ten runs. Lower- and upper-limit of the measurement range (LLMR and ULMR) were determined as the concentrations that denotes the limits of the linear range of the assay. Lower limit of detection was calculated as the mean interpolated concentration of 21 blank samples, only containing assay buffer, plus three standard deviations. Upper limit of detection was calculated as the mean interpolated concentration of standard peptide corresponding to the highest concentration of the standard curve minus three standard deviations.

Analyte stability was evaluated for three human serum samples incubated at either 4 or 20 °C for 2, 4, 24, or 48 hours and calculating the percentage recovery of the incubated samples relative to the corresponding control sample kept at -20 °C. Freeze-thaw stability was evaluated by repeatedly freezing and thawing human serum samples for up to 4 cycles and calculating the percentage recovery of the cycled samples relative to the corresponding control samples that underwent a single freeze-thaw cycle.

### Patient samples - Cohort 1

The cohort included 222 cancer samples and 33 healthy samples. It included 20 patients each of pancreatic-, colorectal-, kidney-, stomach-, breast-, bladder-, lung-, melanoma-, head and neck- and prostate-cancer, 19 ovarian cancer patients, 3 liver cancer patients and 33 age matched healthy controls. All cancer samples were obtained from Proteogenex (Los Angeles, CA, USA) and the healthy controls were obtained from BioIVT (Westbury, NY, USA). A summary of the cohort characteristics can be found in Table 1.

**Table 1: Patient demographics of the cohort.**

| | | **Healthy (N=33)** | **Cancer (N=222)** | **Total (N=255)** |
|---|---|---|---|---|
| **Diagnosis** | | | | |
| | Healthy | 33 (100%) | 0 (0%) | 33 (12.9%) |
| | Bladder cancer | 0 (0%) | 20 (9.0%) | 20 (7.8%) |
| | Breast cancer | 0 (0%) | 20 (9.0%) | 20 (7.8%) |
| | Colorectal cancer | 0 (0%) | 20 (9.0%) | 20 (7.8%) |
| | Head & neck cancer | 0 (0%) | 20 (9.0%) | 20 (7.8%) |
| | Kidney cancer | 0 (0%) | 20 (9.0%) | 20 (7.8%) |
| | Liver cancer | 0 (0%) | 3 (1.4%) | 3 (1.2%) |
| | Lung cancer | 0 (0%) | 20 (9.0%) | 20 (7.8%) |
| | Melanoma | 0 (0%) | 20 (9.0%) | 20 (7.8%) |
| | Ovarian cancer | 0 (0%) | 19 (8.6%) | 19 (7.5%) |
| | Pancreatic cancer | 0 (0%) | 20 (9.0%) | 20 (7.8%) |
| | Prostate cancer | 0 (0%) | 20 (9.0%) | 20 (7.8%) |
| | Stomach cancer | 0 (0%) | 20 (9.0%) | 20 (7.8%) |

| **Stages** | | | | |
|---|---|---|---|---|
| | I | 0 (0%) | 7 (3.2%) | 7 (2.7%) |
| | II | 0 (0%) | 49 (22.1%) | 49 (19.2%) |
| | III | 0 (0%) | 93 (41.9%) | 93 (36.5%) |
| | IV | 0 (0%) | 73 (32.9%) | 73 (28.6%) |

| | | **Healthy (N=33)** | **Cancer (N=222)** | **Total (N=255)** |
|---|---|---|---|---|
| | Missing | 33 (100%) | 0 (0%) | 33 (12.9%) |

| **Age (years)** | | | | |
|---|---|---|---|---|
| | Mean (SD) | 57.7 (5.69) | 59.3 (11.2) | 59.1 (10.7) |
| | Median [Min, Max] | 57.0 [49.0, 69.0] | 61.0 [30.0, 87.0] | 60.5 [30.0, 87.0] |
| | Missing | 0 (0%) | 1 (0.5%) | 1 (0.4%) |

| **Sex** | | | | |
|---|---|---|---|---|
| | Male | 21 (63.6%) | 121 (54.5%) | 142 (55.7%) |
| | Female | 12 (36.4%) | 101 (45.5%) | 113 (44.3%) |

| **Ethnicity** | | | | |
|---|---|---|---|---|
| | Black | 13 (39.4%) | 0 (0%) | 13 (5.1%) |
| | Caucasian | 11 (33.3%) | 222 (100%) | 233 (91.4%) |
| | Hispanic | 9 (27.3%) | 0 (0%) | 9 (3.5%) |

| **BMI** | | | | |
|---|---|---|---|---|
| | Mean (SD) | NA (NA) | 26.4 (4.28) | 26.4 (4.28) |
| | Median [Min, Max] | NA [NA, NA] | 25.8 [17.9, 41.4] | 25.8 [17.9, 41.4] |
| | Missing | 33 (100%) | 1 (0.5%) | 34 (13.3%) |

### Statistics - Cohort 1

Comparisons of PRO-C20 levels across groups was done using ordinary one-way ANOVA followed by pair-wise comparisons to the control group using the Dunnett test. Diagnostic accuracy was tested by the area under the receiver operating characteristics (AUROC) curve. A p-value below 0.05 was considered significant. Asterisks in the figures indicate the following significance levels: * p<0.05; ** p<0.01; *** p<0.001; **** p<0.0001.

Statistical analysis and graphs were done in GraphPad Prism (version 9.1.0 for Windows, GraphPad Software, San Diego, California USA, www.graphpad.com) and R version 4.0.4 (R Core Team (2021), R Foundation for Statistical Computing, Vienna, Austria, https://www.R-project.org).

### Patient Samples - Cohort 2

The cohort included 20 healthy donors and 36 patients with pancreas ductal adenocarcinoma (PDAC). All patients were from the Danish BIOPAC study "BIOmarkers in patients with Pancreatic Cancer" (NCT03311776). Patients were recruited from six Danish hospitals from December 2008 until September 2017. PC patients had histologically confirmed tumors. The PDAC patients were treated with various types of chemotherapy according to national guidelines (www.gicancer.dk). The study was carried out in accordance with the recommendations of the Danish Regional Committee on Health Research Ethics. The BIOPAC protocol was approved by the Danish Regional Committee on Health Research Ethics (VEK ref. KA-20060113) and the Data Protection Agency (j.nr. 2006-41-6848). Blood (serum) samples were obtained at the time of diagnosis or before operation. All subjects gave written informed consent in accordance with the Declaration of Helsinki. Table 2 summarizes the characteristics of the cohort.

**Table 2: Patient demographics of the cohort.**

| **Characteristic** | | | **Healthy**, N = 20 | **PDAC**, N = 36 |
|---|---|---|---|---|
| | **Stage, n (%)** | | | |
| | | 1b | - | 3 (8.3) |
| | | 2a | - | 3 (8.3) |
| | | 2b | - | 11 (31) |
| | | 4 | - | 19 (53) |
| | **Age, Mean (SD)** | | 57 (6) | 66 (8) |

| | **Sex, n (%)** | | | |
|---|---|---|---|---|
| | | Female | 10 (50) | 17 (47) |
| | | Male | 10 (50) | 19 (53) |
| | **Diabetes, n (%)** | | - | 7 (19) |

| | **Tobacco, n (%)** | | | |
|---|---|---|---|---|
| | | Ever | - | 23 (64) |
| | | Never | - | 13 (36) |

### Statistics - Cohort 2

The Wilcoxon test was used to compare PRO-C20 levels between healthy and PDAC samples. Diagnostic accuracy of PRO-C20 was tested using the area under the receiver operating characteristic curve (AUC). In addition, sensitivity, specificity, positive predictive value, and negative predictive value were determined at the PRO-C20 cut-off value where the Youden-index was maximized. The 75th percentile of PRO-C20 levels (i.e. a PRO-C20 level of 2.59 nM)in PDAC samples was used as a cut point to define a group with high PRO-C20 levels. The association of high PRO-C20 levels and overall survival was evaluated using a Kaplan Meier curve with a log-rank test. In addition, multivariate Cox regression analysis evaluated the associated of high PRO-C20 levels when corrected for the presence of metastasis as well as age and sex. The following significance levels are indicated by asterisks: **** p<0.0001.

### Results

### PRO-C20 ELISA development

Optimizations to the ELISA protocol included the best time and temperature of incubation, choice of assay buffer and concentrations of kit components. The settings were chosen based on which gave the best sensitivity in human serum whilst upholding the technical requirements outlined below. The chosen format for the ELISA was competitive, so the specificity of the assay was evaluated by the ability of different peptides to compete for binding to the monoclonal antibody. The set of peptides included the standard peptide, corresponding to the C-terminus of type XX collagen (RHLEGRGEPGAVGQMGSPGQQGASTQGLWE (SEQ ID NO: 6)); an elongated peptide, corresponding to the C-terminus epitope with an extra amino acid (QGASTQGLWES (SEQ ID NO: 2)); a truncated peptide, with an amino acid less (QGASTQGLW (SEQ ID NO: 3)); a non-sense standard peptide, corresponding to an unrelated epitope (SHAHQRTGGN (SEQ ID NO: 8)); and lastly, a non-sense coater peptide (Biotin-SHAHQRTGGN (SEQ ID NO: 9)).

Only the standard peptide dose-dependently inhibited the signal in a meaningful way - the elongated and truncated had minimal competition even at high concentrations (Figure 1). A 10 amino-acid long variant of the standard peptide (QGASTQGLWE (SEQ ID NO: 1)) was also tested and had the exact same behavior as the 30 amino-acid variant (data not shown). The non-sense coater peptide resulted in no detectable signal, as expected. This setup confirms that the monoclonal antibody is specific towards the epitope of interest at the type XX collagen C-terminus.

Other aspects of the technical validation are summarized in Table 3.

**Table 3: Summary of the technical validation of PRO-C20.**

| **Test** | **Result** |
|---|---|
| IC50 | 5.59 ng/mL |
| Measurement range | 0.84-27.3 ng/mL |
| Detection range | 0.602-45.9 ng/mL |
| Dilution recovery of human serum (undiluted to 1:2) | 86.8 % |
| Spiking recovery of peptide in serum | 101.3 % |
| Spiking recovery of serum in serum | 95.5 % |
| Hemoglobin interference recovery, low/high conc. | 104.9/103.7% |
| Lipids interference recovery, low/high conc. | 103.2/109.2% |
| Biotin interference recovery, low/high conc. | 98.1/85.4% |
| Inter-assay variation | 5.4 % |
| Intra-assay variation | 5.9 % |
| Analyte stability (48 hrs 4 °C/48 hrs 20 °C) | 89.5/87.1% |
| Freeze-thaw stability up to four cycles | 89.8 % |

Linearity of dilution or parallelism was accepted from undiluted down to 1:2 dilution. At 1:4 dilution the recovery of human serum samples dropped below the acceptance limit of 80% analyte recovery. Accuracy testing using spiking recovery tests revealed excellent recovery of the standard peptide in human serum with a recovery of 101 %. The same was true with matrix-in-matrix spiking where a spiking of analyte from a human serum sample into another separate human serum sample resulted in a recovery of 95 %. Interference from commonly interfering substances was not observed, with recoveries within 15 % even for the highest concentrations of biotin. Assay variation was excellent at approximately 6% for both inter- and intra-assay variation. Analyte stability was evaluated for up to 48 hours at either 4 or 20 °C and recoveries were within 15 %. Stability following four freeze-thaw cycles was also good with a recovery of 90 %.

### PRO-C20 in serum of cancer patients (Cohort 1)

PRO-C20 levels were significantly elevated in all cancers compared to the healthy controls (Figure 2). PRO-C20 proved excellent at discriminating between healthy and cancer as evidenced by the AUROC values (Table 4).

**Table 4: Area under the ROC curve (AUROC) for the comparison between cancers and controls.**

| **Cancer type** | **AUROC** |
|---|---|
| Bladder cancer | 0.858 |
| Breast cancer | 0.871 |
| Colorectal cancer | 0.897 |
| Head & neck cancer | 0.762 |
| Kidney cancer | 0.885 |
| Lung cancer | 0.921 |
| Melanoma | 0.761 |
| Ovarian cancer | 0.802 |
| Pancreatic cancer | 0.823 |
| Prostate cancer | 0.789 |
| Stomach cancer | 0.826 |

PRO-C20 was particularly good at discriminating between lung cancer and healthy controls with an AUROC of 0.92. Overall, these results suggest that circulating levels of PRO-C20, and hence type XX collagen, are elevated in multiple different cancer types.

### PRO-C20 in serum of PDAC patients (Cohort 2)

Quantification of PRO-C20 in serum of healthy controls (n = 20) and patients with PDAC (n = 36) revealed elevated levels of PRO-C20 in PDAC compared to healthy controls (Figure 3). PRO-C20 had good diagnostic accuracy as shown by the AUC, sensitivity and specificity values (Table 5). For patients with PDAC, high PRO-C20 levels (above 2.59 nM) was associated with poor overall survival (Figure 4). And high PRO-C20 levels were an independent predictor of survival after adjusting for age, sex and the presence of metastasis (Table 6).

**Table 5: AUC, sensitivity and specificity values for the comparison between PDAC and controls.**

| **Positive classifier** | **Negative classifier** | **AUC** | **Cutoff** | **Youden** | **Sensitivity** | **Specificity** | **PPV** | **NPV** |
|---|---|---|---|---|---|---|---|---|
| PDAC | Healthy | 0.92 | 1.71 | 0.77 | 0.97 | 0.80 | 0.90 | 0.94 |

**Table 6: Multivariate Cox regression analysis**

| **Multivariate Cox Regression** | | | |
|---|---|---|---|
| **Characteristic** | | **HR (95% CI)***¹* | **p-value** |
| | PRO-C20: Above 2.59 nM | 4.25 (1.52 to 11.9) | 0.006 |
| | Metastasis: Yes | 5.00 (1.93 to 13.0) | <0.001 |
| | Age: Continuous | 1.08 (1.01 to 1.15) | 0.025 |
| | Sex: Male | 2.71 (1.13 to 6.48) | 0.025 |
| n = 36; N events = 25; R² = 0.549; c-index = 0.808; c-index SE = 0.036 | | | |
| *¹* HR = Hazard Ratio, CI = Confidence Interval | | | |

### Conclusion

In this study, an ELISA to quantify the presence of type XX collagen in blood was successfully developed, optimized, and validated. The PRO-C20 ELISA was technically robust, accurate and sensitive. The levels of circulating type XX collagen could be assessed in the serum of cancer patients and healthy controls, with levels of PRO-C20 being significantly higher in all cancers tested compared to healthy controls. In the serum from pancreas ductal adenocarcinoma (PDAC) patients, high levels of PRO-C20 were furthermore found to be associated with poor overall survival rates.

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used to mean 'including or consisting of'. No acknowledgement of any prior published document herein should be taken to be an admission or representation that the teaching thereof was common general knowledge in Australia or elsewhere at the date hereof.

### References

1. Sung, H.; Ferlay, J.; Siegel, R.L.; Laversanne, M.; Soerjomataram, I.; Jemal, A.; Bray, F. Global cancer statistics 2020: GLOBOCAN estimates of incidence and mortality worldwide for 36 cancers in 185 countries. CA. Cancer J. Clin. 2021, doi:10.3322/caac.21660.
2. Marrugo-Ramírez, J.; Mir, M.; Samitier, J. Blood-Based Cancer Biomarkers in Liquid Biopsy: A Promising Non-Invasive Alternative to Tissue Biopsy. Int. J. Mol. Sci. 2018, 19, 2877, doi:10.3390/ijms19102877.
3. Walker, C.; Mojares, E.; del Río Hernéndez, A. Role of Extracellular Matrix in Development and Cancer Progression. Int. J. Mol. Sci. 2018, 19, 3028, doi:10.3390/ijms19103028.
4. Xu, S.; Xu, H.; Wang, W.; Li, S.; Li, H.; Li, T.; Zhang, W.; Yu, X.; Liu, L. The role of collagen in cancer: from bench to bedside. J. Transl. Med. 2019, 17, 309, doi:10.1186/s12967-019-2058-1.
5. Nissen, N.I.; Karsdal, M.; Willumsen, N. Collagens and Cancer associated fibroblasts in the reactive stroma and its relation to Cancer biology. J. Exp. Clin. Cancer Res. 2019, 38, 115, doi:10.1186/s13046-019-1110-6.
6. Ioachim, E.; Charchanti, A.; Briasoulis, E.; Karavasilis, V.; Tsanou, H.; Arvanitis, D.L.; Agnantis, N.J.; Pavlidis, N. Immunohistochemical expression of extracellular matrix components tenascin, fibronectin, collagen type IV and laminin in breast cancer: Their prognostic value and role in tumour invasion and progression. Eur. J. Cancer 2002, 38, 2362-2370, doi:10.1016/S0959-8049(02)00210-1.
7. Öhlund, D.; Franklin, O.; Lundberg, E.; Lundin, C.; Sund, M. Type IV collagen stimulates pancreatic cancer cell proliferation, migration, and inhibits apoptosis through an autocrine loop. BMC Cancer 2013, 13, doi:10.1186/1471-2407-13-154.
8. Koch, M.; Foley, J.E.; Hahn, R.; Zhou, P.; Burgeson, R.E.; Gerecke, D.R.; Gordon, M.K. alpha 1(Xx) collagen, a new member of the collagen subfamily, fibril-associated collagens with interrupted triple helices. J. Biol. Chem. 2001, 276, 23120-23126, doi:10.1074/jbc.M009912200.
9. Manzo, G. Similarities between embryo development and cancer process suggest new strategies for research and therapy of tumors: A new point of view. Front. Cell Dev. Biol. 2019, 7, doi:10.3389/fcell.2019.00020.
10. Wong, A.Y.; Whited, J.L. Parallels between wound healing, epimorphic regeneration and solid tumors. Dev. 2020, 147.
11. Huang, C.-C.; Tu, S.-H.; Lien, H.-H.; Jeng, J.-Y.; Huang, C.-S.; Huang, C.-J.; Lai, L.-C.; Chuang, E.Y. Concurrent gene signatures for han chinese breast cancers. PLoS One 2013, 8, e76421, doi:10.1371/journal.pone.0076421.
12. Ishihara, E.; Takahashi, S.; Fukaya, R.; Ohta, S.; Yoshida, K.; Toda, M. Identification of KLRC2 as a candidate marker for brain tumor-initiating cells. Neurol. Res. 2019, 41, 1043-1049, doi:10.1080/01616412.2019.1672390.
13. Vitanza, N.A.; Biery, M.C.; Myers, C.; Ferguson, E.; Zheng, Y.; Girard, E.J.; Przystal, J.M.; Park, G.; Noll, A.; Pakiam, F.; et al. Optimal therapeutic targeting by HDAC inhibition in biopsy-derived treatment-naive diffuse midline glioma models. Neuro. Oncol. 2021, 23, 376-386, doi:10.1093/neuonc/noaa249.
14. Gorlov, I.P.; Byun, J.; Gorlova, O.Y.; Aparicio, A.M.; Efstathiou, E.; Logothetis, C.J. Candidate pathways and genes for prostate cancer: a meta-analysis of gene expression data. BMC Med. Genomics 2009, 2, 48, doi:10.1186/1755-8794-2-48.
15. Kehlet, S.N.; Manon-Jensen, T.; Sun, S.; Brix, S.; Leeming, D.J.; Karsdal, M.A.; Willumsen, N. A fragment of SPARC reflecting increased collagen affinity shows pathological relevance in lung cancer - implications of a new collagen chaperone function of SPARC. Cancer Biol. Ther. 2018, 19, 904-912, doi:10.1080/15384047.2018.1480887.

## Claims

1. A monoclonal antibody that specifically binds to a C-terminus amino acid sequence QGASTQGLWE (SEQ ID NO: 1).

2. A monoclonal antibody as claimed in claim 1, wherein the monoclonal antibody does not specifically bind to an elongated version of said C-terminus amino acid sequence which is QGASTQGLWES (SEQ ID NO: 2).

3. A monoclonal antibody as claimed in claim 1 or 2, wherein the monoclonal antibody does not specifically bind to a truncated version of said C-terminus amino acid sequence which is QGASTQGLW (SEQ ID NO: 3).

4. A monoclonal antibody as claimed in any one of claims 1 to 3, wherein the monoclonal antibody is raised against a synthetic peptide having the C-terminus amino acid sequence QGASTQGLWE (SEQ ID NO: 1).

5. A method of immunoassay for detecting and/or monitoring cancer in a patient and/or assessing the severity of a cancer in a patient, the method comprising:
i) contacting a sample from a patient with a monoclonal antibody of any one of claims 1 to 4 and
ii) detecting and determining the amount of binding between said monoclonal antibody and peptides in the sample.

6. The method of claim 5, wherein the method further comprises:
iii) correlating said amount of binding of said monoclonal antibody as determined in step (ii) with values associated with normal healthy subjects, and/or with values associated with known disease severity, and/or with values obtained from said patient at a previous time point, and/or with a predetermined cut-off value.

7. The method of claim 5 or 6, wherein the cancer is bladder cancer, breast cancer, colorectal cancer, head and neck cancer, kidney cancer, liver cancer, lung cancer, melanoma, ovarian cancer, pancreatic cancer, prostate cancer, or stomach cancer.

8. The method of any one of claims 5 to 7, wherein the sample is a biofluid sample selected from blood, serum or plasma.

9. The method of any one of claims 5 to 8, wherein the immunoassay is a competition assay or a sandwich assay.

10. The method of any one of claims 5 to 9, wherein the immunoassay is a radioimmunoassay or an enzyme-linked immunosorbent assay.

11. An immunoassay kit comprising a monoclonal antibody of any one of claims 1 to 4, and at least one of;
- a streptavidin coated well plate;
- a biotinylated peptide Biotin-L- QGASTQGLWE (SEQ ID NO: 4), wherein L is an optional linker;
- a secondary antibody for use in a sandwich immunoassay;
- a calibrator protein comprising the C-terminus amino acid sequence QGASTQGLWE (SEQ ID NO: 1);
- an antibody biotinylation kit;
- an antibody HRP labelling kit;
- an antibody radiolabelling kit; and
- an assay visualisation kit.

## Patentansprüche

1. Ein monoklonaler Antikörper, der spezifisch an eine C-terminale Aminosäuresequenz QGASTQGLWE (SEQ ID NR.: 1) bindet.

2. Monoklonaler Antikörper nach Anspruch 1, wobei der monoklonale Antikörper nicht spezifisch an eine verlängerte Version der C-terminalen Aminosäuresequenz bindet, die QGASTQGLWES (SEQ ID NR.: 2) ist.

3. Monoklonaler Antikörper nach Anspruch 1 oder 2, wobei der monoklonale Antikörper nicht spezifisch an eine verkürzte Version der C-terminalen Aminosäuresequenz bindet, die QGASTQGLW (SEQ ID NR.: 3) ist.

4. Monoklonaler Antikörper nach einem der Ansprüche 1 bis 3, wobei der monoklonale Antikörper gegen ein synthetisches Peptid, aufweisend die C-terminale Aminosäuresequenz QGASTQGLWE (SEQ ID NR.: 1) gebildet ist.

5. Immunoassay-Verfahren zum Nachweisen und/oder Überwachen von Krebs bei einem Patienten und/oder zum Beurteilen des Schweregrads eines Krebses bei einem Patienten, das Verfahren umfassend:
i) Inkontaktbringen einer Probe eines Patienten mit einem monoklonalen Antikörper nach einem der Ansprüche 1 bis 4 und
ii) Nachweisen und Bestimmen des Ausmaßes der Bindung zwischen dem monoklonalen Antikörper und Peptiden in der Probe.

6. Verfahren nach Anspruch 5, das Verfahren ferner umfassend:
iii) Korrelieren der in Schritt (ii) bestimmten Bindungsmenge des monoklonalen Antikörpers mit Werten, die mit normalen gesunden Probanden assoziiert sind, und/oder mit Werten, die mit einem bekannten Schweregrad der Erkrankung assoziiert sind, und/oder mit Werten, die bei dem Patienten zu einem früheren Zeitpunkt erhalten wurden, und/oder mit einem vorbestimmten Grenzwert.

7. Verfahren nach Anspruch 5 oder 6, wobei der Krebs Blasenkrebs, Brustkrebs, kolorektaler Krebs, Kopf- und Halskrebs, Nierenkrebs, Leberkrebs, Lungenkrebs, Melanom, Eierstockkrebs, Bauchspeicheldrüsenkrebs, Prostatakrebs oder Magenkrebs ist.

8. Verfahren nach einem der Ansprüche 5 bis 7, wobei die Probe eine Bioflüssigkeitsprobe ist, ausgewählt aus Blut, Serum oder Plasma.

9. Verfahren nach einem der Ansprüche 5 bis 8, wobei der Immunoassay ein Wettbewerbs-Assay oder ein Sandwich-Assay ist.

10. Verfahren nach einem der Ansprüche 5 bis 9, wobei der Immunoassay ein Radioimmunoassay oder ein enzymgekoppelter Immunadsorptionsassay ist.

11. Immunoassay-Kit, umfassend einen monoklonalen Antikörper nach einem der Ansprüche 1 bis 4 und mindestens einen der folgenden Bestandteile;
- eine mit Streptavidin beschichtete Well-Plate;
- ein biotinyliertes Peptid Biotin-L-QGASTQGLWE (SEQ ID NR.: 4), wobei L ein optionaler Linker ist;
- einen sekundären Antikörper zur Verwendung in einem Sandwich-Immunoassay;
- ein Kalibratorprotein, das die C-terminale Aminosäuresequenz QGASTQGLWE (SEQ ID NR.: 1) umfasst;
- ein Antikörper-Biotinylierungs-Kit;
- ein Antikörper-HRP-Markierungs-Kit;
- ein Antikörper-Radiomarkierungskit; und
- ein Assay-Visualisierungs-Kit.

## Revendications

1. Anticorps monoclonal qui se lie spécifiquement à une séquence d'acides aminés C-terminale QGASTQGLWE (SEQ ID NO : 1).

2. Anticorps monoclonal selon la revendication 1, l'anticorps monoclonal ne se liant pas spécifiquement à une version allongée de ladite séquence d'acides aminés C-terminale qui est QGASTQGLWES (SEQ ID NO : 2).

3. Anticorps monoclonal selon la revendication 1 ou 2, l'anticorps monoclonal ne se liant pas spécifiquement à une version tronquée de ladite séquence d'acides aminés C-terminale qui est QGASTQGLW (SEQ ID NO : 3).

4. Anticorps monoclonal selon l'une quelconque des revendications 1 à 3, l'anticorps monoclonal étant dirigé contre un peptide synthétique présentant la séquence d'acides aminés C-terminale QGASTQGLWE (SEQ ID NO : 1).

5. Procédé de dosage immunologique pour détecter et/ou surveiller un cancer chez un patient et/ou évaluer la gravité d'un cancer chez un patient, le procédé comprenant :
i) la mise en contact d'un échantillon provenant d'un patient avec un anticorps monoclonal selon l'une quelconque des revendications 1 à 4 et
ii) la détection et la détermination de la quantité de liaison entre ledit anticorps monoclonal et des peptides dans l'échantillon.

6. Procédé selon la revendication 5, le procédé comprenant en outre :
iii) la corrélation de ladite quantité de liaison dudit anticorps monoclonal telle que déterminée à l'étape (ii) avec des valeurs associées à des sujets sains normaux et/ou avec des valeurs associées à une gravité connue de maladie et/ou avec des valeurs obtenues à partir dudit patient à un moment antérieur et/ou avec une valeur seuil statistique prédéterminée.

7. Procédé selon la revendication 5 ou 6, dans lequel le cancer est un cancer de la vessie, un cancer du sein, un cancer colorectal, un cancer de la tête et du cou, un cancer du rein, un cancer du foie, un cancer du poumon, un mélanome, un cancer de l'ovaire, un cancer du pancréas, un cancer de la prostate ou un cancer de l'estomac.

8. Procédé selon l'une quelconque des revendications 5 à 7, dans lequel l'échantillon est un échantillon de fluide biologique choisi parmi le sang, le sérum ou le plasma.

9. Procédé selon l'une quelconque des revendications 5 à 8, dans lequel le dosage immunologique est un dosage par compétition ou un dosage de type sandwich.

10. Procédé selon l'une quelconque des revendications 5 à 9, dans lequel le dosage immunologique est un dosage radio-immunologique ou un dosage d'immunoabsorption enzymatique.

11. Trousse de dosage immunologique comprenant un anticorps monoclonal selon l'une quelconque des revendications 1 à 4, et au moins l'un parmi :
- une plaque à puits revêtue de streptavidine ;
- un peptide biotinylé Biotine-L-QGASTQGLWE (SEQ ID NO: 4), où L est un lieur facultatif ;
- un anticorps secondaire pour une utilisation dans un dosage de type sandwich ;
- une protéine étalon comprenant la séquence d'acides aminés C-terminale QGASTQGLWE (SEQ ID NO: 1) ;
- un kit de biotinylation d'anticorps ;
- un kit de marquage HRP d'anticorps ;
- un kit de radiomarquage d'anticorps ; et
- un kit de visualisation de dosage.
